# EUROPEAN PATENT SPECIFICATION

(11) **EP 4 260 884 B1**
(45) Date of publication and mention of the grant of the patent: **10.06.2026**
(21) Application number: 23167805.3
(22) Date of filing: 13.04.2023
(51) Int. Cl.: A61M 1/16, A61B 50/00, A61B 50/22, A61B 50/30, A61B 50/31

(54) **BIOMEDICAL EQUIPMENT AND CASING FOR THE TRANSPORT OF A BIOMEDICAL UNIT**
BIOMEDIZINISCHE AUSRÜSTUNG UND GEHÄUSE FÜR DEN TRANSPORT EINER BIOMEDIZINISCHEN EINHEIT
EQUIPEMENT BIOMÉDICAL ET BOÎTIER POUR LE TRANSPORT D'UNE UNITÉ BIOMÉDICALE

(30) Priority: 15.04.2022 IT 202200007595
(43) Date of publication of application: 18.10.2023
(73) Proprietor: Eurosets S.r.l., 41036 Medolla (MO) (IT)
(72) Inventor: PETRALIA, Antonio, 41036 Medolla (MO) (IT); FONTANILI, Paolo, 41036 Medolla (MO) (IT)
(74) Representative: Zoli, Filippo

(56) References cited:
- DE-A1- 102020 005 197
- US-A1- 2011 208 107
- US-A1- 2022 080 090
- US-B1- 10 455 923

## Description

### Technical Field

The present invention relates to a biomedical equipment and casing for the transport of a biomedical unit.

### Background Art

As is well known, during certain surgical operations, during which the patient's heart functions are temporarily suspended, extracorporeal blood circuits are made using the so-called "heart-lung" machines.

Heart-lung machines comprise a number of devices, including a filtering device (also called a "venous reserve") adapted to filter the incoming blood from the patient, a heat exchanger adapted to adjust the temperature of the blood exiting the filtering device and an oxygenator adapted to provide the proper oxygen supply to the blood intended to be fed back into the patient. Specifically, the incoming blood from the patient is sent to the oxygenator by means of a related pumping assembly, which generally comprises a centrifugal pump of the dragging or magnetic levitation type.

A number of parameters need to be monitored and controlled during extracorporeal circulation, including the number of revolutions of the rotor element and the flow rate of blood sent to the oxygenator.

Appropriately, during extracorporeal circulation, equipment is used that has a load-bearing frame which supports the oxygenator and the pumping assembly and is provided with a processing unit to control and command the aforementioned parameters.

This equipment is generally used in both hospital and out-of-hospital settings, since extracorporeal circulation is also applied under emergency situations, such as in roadside rescue, during which there is a need to adequately support the various devices in a dynamic situation and in a flexible manner.

The known type of equipment for the support of the biomedical devices, particularly for supporting the oxygenator and the relevant pumping assembly, does however have some drawbacks.

They, in fact, although small in size compared to equipment used only in the hospital setting, do not allow for easy and practical movement of the oxygenator and the relevant pumping assembly in out-of-hospital emergency situations wherein the patient is in places that are difficult to reach by emergency vehicles, that is, where there is a need to adequately support the various devices in a dynamic situation and in a flexible manner.

It follows, therefore, that generally the load-bearing frame is moved separately from the devices themselves, thus requiring multiple operators to transport the various components. This also results in the need, once at the rescue site, to correctly position the biomedical devices on the relevant load-bearing frame and to connect them to each other before being able to activate the extracorporeal circulation. As can be easily guessed, this inevitably results in longer intervention times.

DE102020005197A1 discloses a packaging system for medical tubing sets comprising a support element having recesses for accommodating an oxygenator and a medical tube set for extracorporeal therapy.

US10455923B1 discloses an enteral feeding system equipment comprising a load-bearing frame provided with support means of a feeding machine and one containment casing for containing the equipment.

### Description of the Invention

The main aim of the present invention is to devise a piece of biomedical equipment which allows the easy and convenient transport of a biomedical device together with the relevant load-bearing frame.

Within this aim, one object of the present invention is to devise a piece of equipment which can be carried by a single operator and which, once at the rescue site, allows immediate use of the transported biomedical device. Another object of the present invention is to ensure the safe transport of the biomedical device in order to reduce as much as possible the risk that it may be damaged while being displaced to reach the rescue site.

Another object of the present invention is to devise a biomedical piece of equipment which allows the aforementioned drawbacks of the prior art to be overcome within the framework of a simple, rational, easy and effective to use as well as cost-effective solution.

The aforementioned objects are achieved by this biomedical piece of equipment according to claim 1.

The aforementioned objects are also achieved by this casing for the transport of a biomedical unit according to claim 10.

### Brief Description of the Drawings

Other characteristics and advantages of the present invention will become more apparent from the description of a preferred, but not exclusive embodiment of a biomedical piece of equipment and a casing for the transport of a biomedical unit, illustrated by way of an indicative, yet non-limiting example in the accompanying tables of drawings in which:

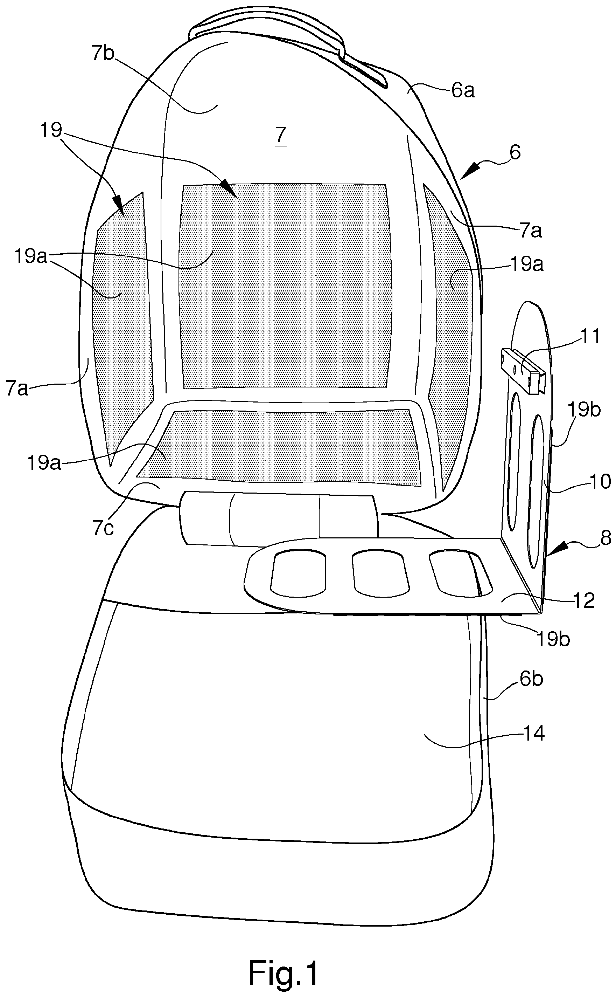

Figure 1 is an axonometric view of a casing according to the invention;

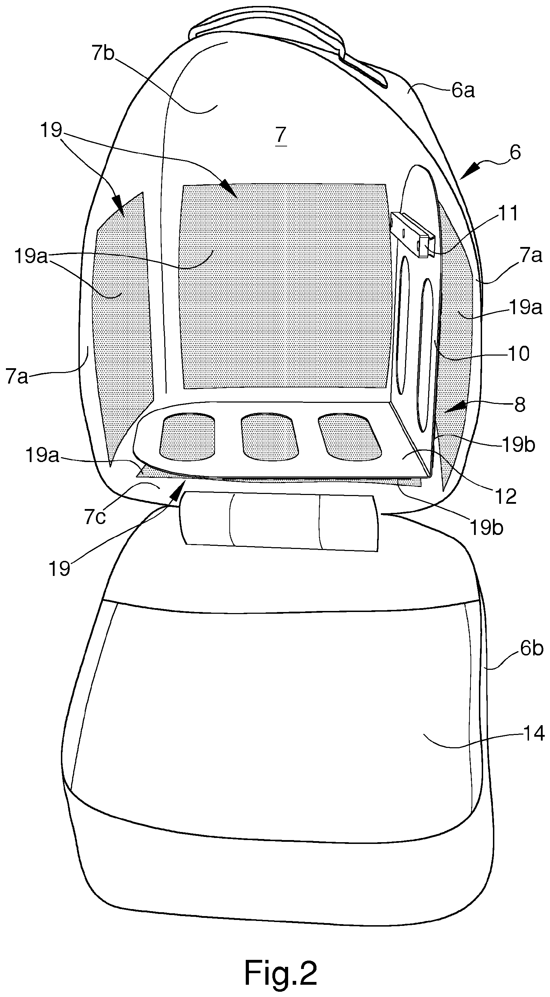

Figure 2 is an axonometric view of a biomedical piece of equipment according to the invention.

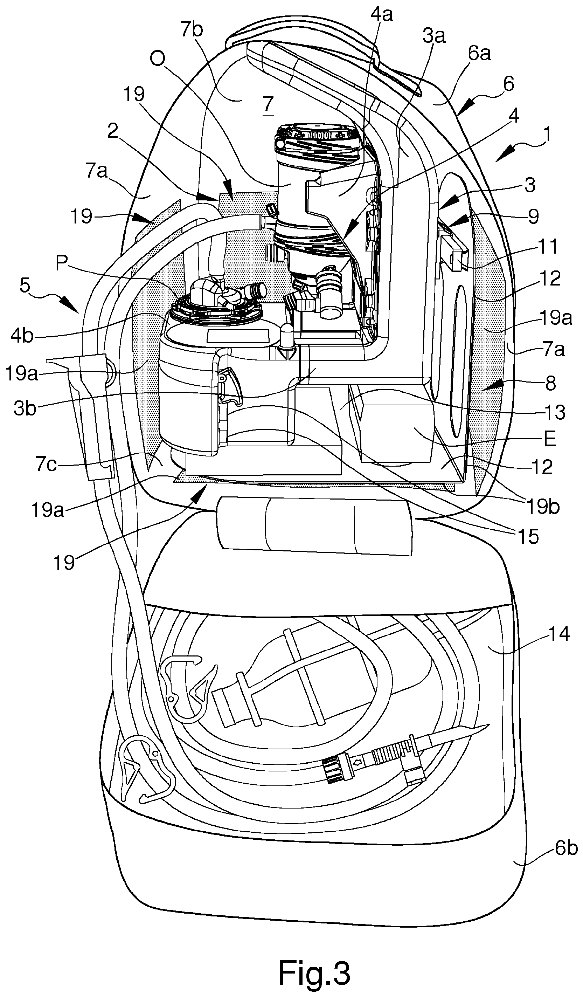

Figure 3 is an axonometric view of a biomedical piece of equipment according to the invention.

### Embodiments of the Invention

With particular reference to these figures, reference numeral 1 globally denotes a biomedical piece of equipment, particularly for extracorporeal circulation.

The piece of equipment 1 comprises a biomedical unit 2 provided with a load-bearing frame 3, with supporting means 4 of at least one biomedical device O,P associated with the load-bearing frame 3 and at least one biomedical device O,P associated with the supporting means 4.

More particularly, the supporting means 4 comprise first supporting means 4a and second supporting means 4b and the biomedical device O,P comprises at least one oxygenator O associated with the first supporting means 4a and at least one centrifugal pump P associated with the second supporting means 4b. The oxygenator O is adapted to provide the proper supply of oxygen to the blood intended to be fed back into the patient and to remove carbon dioxide from the blood itself.

Appropriately, the first supporting means 4a comprise a first seat intended to receive the oxygenator O. More specifically, the first seat is shaped so as to receive the oxygenator O by slotting in and has at least one abutment surface adapted to support the oxygenator itself.

Preferably, the first supporting means 4a are associated with the load-bearing frame 3 in a removable manner.

The centrifugal pump P is, e.g., of the magnetic driving type, i.e., provided with at least one rotor element and with motor means for the rotational drive of the rotor element (the rotor element and the motor means are not visible in detail in the figures). Advantageously, the second supporting means 4b comprise at least one containment body associated with the load-bearing frame 3 and adapted to support the centrifugal pump P.

The rotor element is contained within a hollow body and the motor means comprise a containment body within which a stator element is inserted and adapted to generate a magnetic field to command the rotor element in rotation around a relevant axis, without contact. The controlling methods of the rotation of the rotor element, although not covered by the present invention, are widely known to the engineer in the field.

Specifically, the stator element comprises a plurality of windings intended to be traversed by the electric current for the formation of one or more magnetic fields adapted to interact with the rotor element to lift and set it in rotation around the relevant axis.

**In** more detail, the second supporting means 4b define a second housing seat intended to receive the motor means of the centrifugal pump P. The motor means of the centrifugal pump P are then housed within the second seat and the rotor element is connectable to the motor means in a removable manner.

Next, the biomedical unit 2 comprises a fluid-operated circuit 5 for the blood circulation connected to the oxygenator O and to the centrifugal pump P. Appropriately, the load-bearing frame 3 comprises one or more electrical connectors 15 which can be connected to the electrical user points.

According to the invention, the piece of equipment 1 comprises at least one containment casing 6 for containing the biomedical unit 2 defining at least one housing chamber 7 of the load-bearing frame 3 and provided with anchoring means 8 arranged within the housing chamber 7. The load-bearing frame 3 is provided with gripping means 9 adapted to operate in conjunction with the anchoring means 8 to clamp the position of the biomedical unit 2 within the housing chamber 7.

Preferably, the casing 6 is of the flexible type and the anchoring means 8 are of the rigid type.

In more detail, the housing chamber 7 is bounded by at least one side wall 7a, by a front wall 7b and by a base wall 7c which are arranged transverse to each other. As visible from the figures, the side wall 7a and the front wall 7b are arranged, in use, vertically, while the base wall 7c is arranged in use horizontally. Appropriately, the casing 6 has two side walls 7a arranged facing each other.

Advantageously, the anchoring means 8 comprise at least a first slab-shaped element 10 of the rigid type, which is associated with the casing 6 and at least one anchoring element 11 having an elongated shape associated with the first slab-shaped element 10 and facing towards the inside of the housing chamber 7. The gripping means 9 are adapted to intercept the anchoring element 11 to lock the biomedical unit 2 to the first slab-shaped element 10 and, therefore, to the casing 6.

More particularly, the anchoring element 11 is of the type of a bar having an elongated shape and the gripping means 9 are of the gripper type and are movable between an open position, in order to allow the insertion/extraction of the anchoring element 11 inside them, and a closed position, wherein they interact with the anchoring element 11 in order to prevent the mutual displacement thereof.

Appropriately, the first slab-shaped element 10 is arranged, in use (i.e., during the normal use of the equipment 1), substantially vertically, while the anchoring element 11 is arranged substantially horizontally.

Preferably, the anchoring means 8 comprise at least a second slab-shaped element 12 of the rigid type, associated with the first slab-shaped element 10 and arranged transversely with respect thereto, to substantially define an L. The load-bearing frame 3 comprises a first portion 3a supporting the gripping means 9 and a second portion 3b, locked together with the first portion 3a and adapted to be arranged in support on the second slab-shaped element 12.

In more detail, the load-bearing frame 3 is shaped in such a way as to define a housing space 13 between the load-bearing frame itself and the second slab-shaped element 12 with the gripping means 9 engaged with the anchoring element 11. The housing space 13 is intended to receive power supply means E, e.g., of the type of a rechargeable battery, connectable to the connectors 15.

Advantageously, the anchoring means 8 are associated with the casing 6 by means of fastening means 19 of the removable type, e.g. through Velcro or the like. The anchoring means 8 can then be removed from the casing 6 according to the specific needs of the case.

More specifically, the fastening means 19 comprise at least a first fastener 19a associated with at least one of the side walls 7a, the front wall 7b and the base wall 7c and, preferably, with each of them, and at least a second fastener 19b associated with the anchoring means 8 and adapted to operate in conjunction with a relevant first fastener 19a, so that the operator can arrange the anchoring means themselves in the position from time to time most appropriate for the individual needs of the case.

More particularly, the first fastener 19a is associated with the base wall 7c and with at least one of the side walls 7a and the front wall 7b, while the second fastener 19b is associated with both the first slab-shaped element 10 and the second slab-shaped element 12. **In** the embodiment shown in the figures, the first fastener 19a is thus associated with the two side walls 7a, the front wall 7b and the base wall 7c, from which it follows that the operator can attach the anchoring means 8 to the casing 6 in three different positions by orienting the load-bearing frame 3 to the right, to the left or to the front of the operator accordingly.

Attaching the anchoring means 8 to one of the side walls 7a and the front wall 7b and, simultaneously, to the base wall 7c allows the anchoring means themselves to be locked together with the casing 6.

Preferably, the fastening means 19 are conformed in a band shape, that is, they extend along two directions transverse to each other so as to allow easy adjustment of the position of the anchoring means 8 with respect to the casing 6. In the preferred embodiment shown in the figures, the casing 6 also comprises a compartment 14 for housing the fluid-operated circuit 5.

More particularly, the casing 6 comprises two parts 6a,6b, of which a first part 6a defining the housing chamber 7 and a second part 6b defining the compartment C. In this embodiment, the casing 6 is of the openable type so as to mutually move the parts 6a and 6b between a closed configuration, wherein the housing chamber 7 and the compartment 14 face each other and are inaccessible from the outside, and an open configuration, wherein the housing chamber 7 and the compartment are spaced apart from each other with respect to the closed configuration and are accessible from the outside.

Appropriately, the casing 6 is of the type of a backpack made of a flexible material and provided with carrying means, such as a handle or shoulder straps intended to be worn by an operator.

The operation of this invention is as follows.

The casing 6 is first arranged in an open configuration so that the two parts 6a and 6b are spaced apart from each other and are accessible from the outside. The anchoring means 8 are then applied to the casing 6, placing them inside the housing chamber 7 so that they are locked therewith.

Next, the biomedical unit 2, wherein the oxygenator O and the centrifugal pump P have already been associated with their respective supporting means 4 and connected to the fluid-operated circuit 5, is also placed inside the housing chamber 7.

More particularly, the load-bearing frame 3 is locked together with the anchoring means 8 due to the engagement of its gripping means 9 with the anchoring element 11. More particularly, the gripper 9 is operated so as to receive the anchoring element 11 inside it and is subsequently brought to the closed configuration so as to clamp the anchoring element itself.

In this way, the biomedical unit 2 is locked together with the anchoring means 8 and, therefore, with the casing 6.

The fluid-operated circuit 5 is then placed inside the compartment and the power supply means E are connected to a relevant electrical connector and arranged inside the housing space 13.

At this point, the casing 6 can be brought to a closed configuration so that it can be easily transported by an operator without the need for auxiliary means. Once the operator arrives at the emergency site, he or she can then open the casing 6 and use the biomedical unit 2, which is already ready for use.

It has in practice been ascertained that the described invention achieves the intended objects and, in particular, the fact is emphasized that the equipment covered by the present invention enables a single operator to conveniently and easily transport a biomedical unit for extracorporeal blood circulation without the need to use mechanical means of transportation, and at the same time allows for ready use of the biomedical unit itself, which is pre-assembled and ready for use.

In particular, the casing allows it to be easily gripped or worn by the operator, and the anchoring means with which it is provided allow the biomedical unit to be kept stable inside it during transport.

Again, the positioning of the fastening means on several walls bounding the housing chamber and separate from each other allows the biomedical unit to be arranged in the most appropriate position for the specific needs of the case, so as to facilitate connection to the patient and access by the operator.

## Claims

1. Biomedical equipment (1) comprising:
- a biomedical unit (2) provided with:
∘ a load-bearing frame (3);
∘ supporting means (4) of at least one biomedical device (O; P) associated with said load-bearing frame (3);
∘ at least one biomedical device (O, P) associated with said supporting means (4);
- at least one containment casing (6) for containing said biomedical unit (2) defining at least one housing chamber (7) of said load-bearing frame (3) and provided with anchoring means (8) arranged within said housing chamber (7), said load-bearing frame (3) being provided with gripping means (9) adapted to operate in conjunction with said anchoring means (8) to clamp the position of said biomedical unit (2) within said housing chamber (7);
**characterized by** the fact that
said biomedical unit (2) is pre-assembled and ready for use,
said supporting means (4) comprise first supporting means (4a) and second supporting means (4b) and said biomedical device (O,P) comprises at least one oxygenator (O) associated with the first supporting means (4a), which comprise a first seat intended to receive the oxygenator (O), and at least one centrifugal pump (P) associated with said second supporting means (4b), the centrifugal pump (P) being of the magnetic driving type and provided with at least one rotor element and with motor means for the rotational drive of the rotor element, where said second supporting means (4b) define a second housing seat intended to receive said motor means of the centrifugal pump (P), said motor means being housed within said second seat and said rotor element being connectable to the motor means in a removable manner,
by the fact that said casing (6) is of the flexible type and said anchoring means (8) are of the rigid type, where said anchoring means (8) comprise at least a first slab-shaped element (10) of the rigid type associated with said containment casing (6) and at least one anchoring element (11) having an elongated shape, associated with said first slab-shaped element (10) and facing towards the inside of said housing chamber (7), said gripping means (9) being adapted to intercept said anchoring element (11)
and by the fact that said anchoring means (8) comprise at least a second slab-shaped element (12) of the rigid type, associated with said first slab-shaped element (10) and arranged transversely with respect thereto, said load-bearing frame (3) comprising a first portion (3a) supporting said gripping means (9) and a second portion (3b), locked together with said first portion (3a), adapted to be arranged in support on said second slab-shaped element (12).

2. Equipment (1) according to claim 1, **characterized by** the fact that said first slab-shaped element (10) is arranged, in use, substantially vertically.

3. Equipment (1) according to claim 1 or 2, **characterized by** the fact that said first slab-shaped element (10) and said second slab-shaped element (12) are locked together with each other to define an L.

4. Equipment (1) according to one or more of the preceding claims, **characterized by** the fact that said load-bearing frame (3) is shaped in such a way as to define a housing space (13) between the load-bearing frame itself and said second slab-shaped element (12) with said gripping means (9) engaged with said anchoring element (11).

5. Equipment (1) according to one or more of the preceding claims, **characterized by** the fact that said gripping means (9) are of the gripper type and are movable between an open position, in order to allow the insertion/extraction of said anchoring element (11) inside them, and a closed position, wherein they interact with said anchoring element (11) in order to prevent the mutual displacement thereof.

6. Equipment (1) according to one or more of the preceding claims, **characterized by** the fact that said casing (6) defines at least two side walls (7a), a front wall (7b) and a base wall (7c) bounding said housing chamber (7), by the fact that said anchoring means (8) are associated with said casing (6) by means of removable fastening means (19), and by the fact that said fastening means (19) comprise at least a first fastener (19a) associated with each of said side walls (7a), with said front wall (7b) and with said base wall (7c) and at least a second fastener (19b) associated with said first slab-shaped element (10) and with said second slab-shaped element (12) and adapted to operate in conjunction with a relevant said first fastener (19a) to lock said anchoring means (8) together with said casing (6).

7. Equipment (1) according to one or more of the preceding claims, **characterized by** the fact that said containment casing (6) is of the type of a backpack made of a flexible material.

8. Equipment (1) according to claim 7, **characterized by** the fact that said biomedical unit (2) comprises a fluid-operated circuit (5) for blood circulation connected to said oxygenator (O) and to said centrifugal pump (P) and by the fact that said containment casing (6) comprises a compartment (14) for housing said fluid-operated circuit (5).

9. Equipment (1) according to claim 8, **characterized by** the fact that said containment casing (6) comprises at least two parts (6a, 6b), of which a first part (6a) defining said housing chamber (7) and a second part (6b) defining said compartment (14), and by the fact that said casing (6) is of the openable type so as to mutually move said parts (6a, 6b) between a closed configuration, wherein said housing chamber (7) and said compartment (14) face each other and are inaccessible from the outside, and an open configuration, wherein said housing chamber (7) and said compartment (14) are spaced apart from each other with respect to said closed configuration and are accessible from the outside.

10. Casing (6) for the transport of a biomedical unit (2) provided with a flexible load-bearing frame (3) and with supporting means (4) of at least one biomedical device (O, P), said casing (6) comprising at least one housing chamber (7) of said load-bearing frame (3) provided with anchoring means (8) engageable with the load-bearing frame itself to clamp the position thereof within said housing chamber (7),
**characterized by** the fact that said casing (6) is of the flexible type and said anchoring means (8) are of the rigid type
and by the fact that said anchoring means (8) comprise at least a first slab-shaped element (10) of the rigid type associated with said containment casing (6) and at least one anchoring element (11) having an elongated shape, associated with said first slab-shaped element (10) and facing towards the inside of said housing chamber (7), said gripping means (9) being adapted to intercept said anchoring element (11) and by the fact that said anchoring means (8) comprise at least a second slab-shaped element (12) of the rigid type, associated with said first slab-shaped element (10) and arranged transversely with respect thereto, said load-bearing frame (3) comprising a first portion (3a) supporting said gripping means (9) and a second portion (3b), locked together with said first portion (3a), adapted to be arranged in support on said second slab-shaped element (12).

## Patentansprüche

1. Biomedizinische Ausrüstung (1), umfassend:
- eine biomedizinische Einheit (2), versehen mit:
∘ einem tragenden Rahmen (3);
∘ Mitteln zum Abstützen (4) mindestens einer biomedizinischen Vorrichtung (O, P), die dem tragenden Rahmen (3) zugeordnet sind;
∘ mindestens einer biomedizinischen Vorrichtung (O, P), die den Mitteln zum Abstützen (4) zugeordnet ist;
- mindestens ein Aufnahmegehäuse (6) zum Aufnehmen der biomedizinischen Einheit (2), das mindestens eine Gehäusekammer (7) des tragenden Rahmens (3) definiert und mit Verankerungsmitteln (8) versehen ist, die innerhalb der Gehäusekammer (7) angeordnet sind, wobei der tragende Rahmen (3) mit Greifmitteln (9) versehen ist, die dazu ausgebildet sind, in Verbindung mit den Verankerungsmitteln (8) zu arbeiten, um die Position der biomedizinischen Einheit (2) innerhalb der Gehäusekammer (7) festzuhalten;
**dadurch gekennzeichnet,**
**dass** die biomedizinische Einheit (2) vormontiert und einsatzbereit ist,
**dass** die Mittel zum Abstützen (4) erste Mittel zum Abstützen (4a) und zweite Mittel zum Abstützen (4b) umfassen und dass die biomedizinische Vorrichtung (O, P) mindestens einen Oxygenator (O), der den ersten Mitteln zum Abstützen (4a) zugeordnet ist, die einen ersten Sitz zur Aufnahme des Oxygenators (O) umfassen, und mindestens eine Zentrifugalpumpe (P), die den zweiten Mitteln zum Abstützen (4b) zugeordnet ist, umfasst, wobei die Zentrifugalpumpe (P) vom magnetisch antreibenden Typ ist und mit mindestens einem Rotorelement und mit Motormitteln für den Drehantrieb des Rotorelements versehen ist, wobei die zweiten Mittel zum Abstützen (4b) einen zweiten Gehäusesitz definieren, der dazu bestimmt ist, die Motormittel der Zentrifugalpumpe (P) aufzunehmen, wobei die Motormittel innerhalb des zweiten Sitzes untergebracht sind und das Rotorelement mit den Motormitteln in einer entfernbaren Weise verbindbar ist,
**dass** das Gehäuse (6) vom flexiblen Typ ist und die Verankerungsmittel (8) vom starren Typ sind, wobei die Verankerungsmittel (8) mindestens ein erstes plattenförmiges Element (10) vom starren Typ, das dem Aufnahmegehäuse (6) zugeordnet ist, und mindestens ein Verankerungselement (11) mit einer länglichen Form umfassen, das dem ersten plattenförmigen Element (10) zugeordnet ist und dem Inneren der Gehäusekammer (7) zugewandt ist, wobei die Greifmittel (9) dazu ausgebildet sind, das Verankerungselement (11) abzufangen,
und **dass** die Verankerungsmittel (8) mindestens ein zweites plattenförmiges Element (12) des starren Typs umfassen, das dem ersten plattenförmigen Element (10) zugeordnet und quer dazu angeordnet ist, wobei der tragende Rahmen (3) einen ersten Abschnitt (3a), der die Greifmittel (9) trägt, und einen zweiten Abschnitt (3b) umfasst, der mit dem ersten Abschnitt (3a) verriegelt ist und dazu ausgebildet ist, als Stütze auf dem zweiten plattenförmigen Element (12) angeordnet zu werden.

2. Ausrüstung (1) nach Anspruch 1, **dadurch gekennzeichnet, dass** das erste plattenförmige Element (10) im Gebrauch im Wesentlichen vertikal angeordnet ist.

3. Ausrüstung (1) nach Anspruch 1 oder 2, **dadurch gekennzeichnet, dass** das erste plattenförmige Element (10) und das zweite plattenförmige Element (12) miteinander verriegelt sind, um ein L zu definieren.

4. Ausrüstung (1) nach einem oder mehreren der vorhergehenden Ansprüche, **dadurch gekennzeichnet, dass** der tragende Rahmen (3) so geformt ist, dass er einen Aufnahmeraum (13) zwischen dem tragenden Rahmen selbst und dem zweiten plattenförmigen Element (12) definiert, wobei die Greifmittel (9) mit dem Verankerungselement (11) in Eingriff stehen.

5. Ausrüstung (1) nach einem oder mehreren der vorhergehenden Ansprüche, **dadurch gekennzeichnet, dass** die Greifmittel (9) vom Greifertyp sind und zwischen einer offenen Position, um das Einsetzen/Entnehmen des Verankerungselements (11) in/aus ihnen zu ermöglichen, und einer geschlossenen Position, in der sie mit dem Verankerungselement (11) zusammenwirken, um deren gegenseitige Verschiebung zu verhindern, beweglich sind.

6. Ausrüstung (1) nach einem oder mehreren der vorhergehenden Ansprüche, **dadurch gekennzeichnet, dass** das Gehäuse (6) mindestens zwei Seitenwände (7a), eine Vorderwand (7b) und eine Bodenwand (7c) definiert, die die Gehäusekammer (7) begrenzen, dass die Verankerungsmittel (8) dem Gehäuse (6) mittels abnehmbarer Befestigungsmittel (19) zugeordnet sind, und dadurch, dass die Befestigungsmittel (19) mindestens ein erstes Befestigungselement (19a), das jeder der Seitenwände (7a), der Vorderwand (7b) und der Bodenwand (7c) zugeordnet ist, und mindestens ein zweites Befestigungselement (19b), das dem ersten plattenförmigen Element (10) und dem zweiten plattenförmigen Element (12) zugeordnet ist, umfassen und dazu ausgebildet sind, in Verbindung mit einem entsprechenden ersten Befestigungselement (19a) zu wirken, um die Verankerungsmittel (8) mit dem Gehäuse (6) zu verriegeln.

7. Ausrüstung (1) nach einem oder mehreren der vorhergehenden Ansprüche, **dadurch gekennzeichnet, dass** das Aufnahmegehäuse (6) von der Art eines aus einem flexiblen Material hergestellten Rucksacks ist.

8. Ausrüstung (1) nach Anspruch 7, **dadurch gekennzeichnet, dass** die biomedizinische Einheit (2) einen fluidbetriebenen Kreislauf (5) für die Blutzirkulation umfasst, der mit dem Oxygenator (O) und der Zentrifugalpumpe (P) verbunden ist, und dass das Aufnahmegehäuse (6) ein Abteil (14) zur Aufnahme des fluidbetriebenen Kreislaufs (5) umfasst.

9. Ausrüstung (1) nach Anspruch 8, **dadurch gekennzeichnet, dass** das Aufnahmegehäuse (6) mindestens zwei Teile (6a, 6b) umfasst, von denen ein erster Teil (6a) die Gehäusekammer (7) und ein zweiter Teil (6b) das Abteil (14) definiert, und dass das Gehäuse (6) von der Art ist, die geöffnet werden kann, um die Teile (6a, 6b) zwischen einer geschlossenen Konfiguration, in der die Gehäusekammer (7) und das Abteil (14) einander zugewandt und von außen unzugänglich sind, und einer offenen Konfiguration, in der die Gehäusekammer (7) und das Abteil (14) in Bezug auf die geschlossene Konfiguration voneinander beabstandet und von außen zugänglich sind, zu bewegen.

10. Gehäuse (6) für den Transport einer biomedizinischen Einheit (2), die mit einem flexiblen tragenden Rahmen (3) und mit Mitteln zum Abstützen (4) von mindestens einer biomedizinischen Vorrichtung (O, P) versehen ist, wobei das Gehäuse (6) mindestens eine Gehäusekammer (7) des tragenden Rahmens (3) umfasst, die mit Verankerungsmitteln (8) versehen ist, die mit dem tragenden Rahmen selbst in Eingriff gebracht werden können, um dessen Position innerhalb der Gehäusekammer (7) festzuhalten, **dadurch gekennzeichnet, dass** das Gehäuse (6) vom flexiblen Typ ist und die Verankerungsmittel (8) vom starren Typ sind, und dadurch, dass die Verankerungsmittel (8) mindestens ein erstes plattenförmiges Element (10) vom starren Typ, das dem Aufnahmegehäuse (6) zugeordnet ist, und mindestens ein Verankerungselement (11) mit einer länglichen Form umfassen, das dem ersten plattenförmigen Element (10) zugeordnet und dem Inneren der Gehäusekammer (7) zugewandt ist, wobei die Greifmittel (9) dazu ausgebildet sind, das Verankerungselement (11) abzufangen, und dadurch, dass die Verankerungsmittel (8) mindestens ein zweites plattenförmiges Element (12) des starren Typs umfassen, das dem ersten plattenförmigen Element (10) zugeordnet und quer zu diesem angeordnet ist, wobei der tragende Rahmen (3) einen ersten Abschnitt (3a), der die Greifmittel (9) trägt, und einen zweiten Abschnitt (3b) umfasst, der mit dem ersten Abschnitt (3a) verriegelt ist und dazu ausgebildet ist, als Stütze auf dem zweiten plattenförmigen Element (12) angeordnet zu werden.

## Revendications

1. - Equipement biomédical (1) comprenant :
- une unité biomédicale (2) comportant :
∘ un cadre porteur de charge (3) ;
∘ des moyens de support (4) d'au moins un dispositif biomédical (O ; P) associés audit cadre porteur de charge (3) ;
∘ au moins un dispositif biomédical (O, P) associé auxdits moyens de support (4) ;
- au moins une enveloppe de confinement (6) pour contenir ladite unité biomédicale (2) définissant au moins une chambre de logement (7) dudit cadre porteur de charge (3) et comportant des moyens d'ancrage (8) disposés à l'intérieur de ladite chambre de logement (7), ledit cadre porteur de charge (3) comportant des moyens de préhension (9) aptes à fonctionner en coopération avec lesdits moyens d'ancrage (8) afin de bloquer la position de ladite unité biomédicale (2) à l'intérieur de ladite chambre de logement (7) ;
**caractérisé par le fait que**
ladite unité biomédicale (2) est préassemblée et prête à l'emploi,
lesdits moyens de support (4) comprennent des premiers moyens de support (4a) et des seconds moyens de support (4b), et ledit dispositif biomédical (O, P) comprend au moins un oxygénateur (O) associé aux premiers moyens de support (4a), qui comprennent un premier siège destiné à recevoir l'oxygénateur (O), et au moins une pompe centrifuge (P) associée auxdits seconds moyens de support (4b), la pompe centrifuge (P) étant du type à entraînement magnétique et comportant au moins un élément rotor et des moyens moteurs pour l'entraînement en rotation de l'élément rotor, lesdits seconds moyens de support (4b) définissent un second siège de logement destiné à recevoir lesdits moyens moteurs de la pompe centrifuge (P), lesdits moyens moteurs étant logés dans ledit second siège et ledit élément rotor étant apte à être relié aux moyens moteurs d'une manière amovible,
**par le fait que** ladite enveloppe (6) est du type souple et lesdits moyens d'ancrage (8) sont du type rigide, lesdits moyens d'ancrage (8) comprenant au moins un premier élément en forme de plaque (10) du type rigide associé à ladite enveloppe de confinement (6) et au moins un élément d'ancrage (11) ayant une forme allongée, associé audit premier élément en forme de plaque (10) et orienté vers l'intérieur de ladite chambre de logement (7), lesdits moyens de préhension (9) étant aptes à intercepter ledit élément d'ancrage (11),
et **par le fait que** lesdits moyens d'ancrage (8) comprennent au moins un second élément en forme de plaque (12) du type rigide, associé audit premier élément en forme de plaque (10) et disposé transversalement par rapport à celui-ci, ledit cadre porteur de charge (3) comprenant une première partie (3a) portant lesdits moyens de préhension (9) et une seconde partie (3b), verrouillée conjointement avec ladite première partie (3a), apte à être disposée en appui sur ledit second élément en forme de plaque (12).

2. - Equipement (1) selon la revendication 1, **caractérisé par le fait que** ledit premier élément en forme de plaque (10) est disposé, en utilisation, sensiblement verticalement.

3. - Equipement (1) selon la revendication 1 ou 2, **caractérisé par le fait que** ledit premier élément en forme de plaque (10) et ledit second élément en forme de plaque (12) sont verrouillés ensemble l'un à l'autre pour définir un L.

4. - Equipement (1) selon l'une ou plusieurs des revendications précédentes, **caractérisé par le fait que** ledit cadre porteur de charge (3) est conformé de manière à définir un espace de logement (13) entre le cadre porteur de charge lui-même et ledit second élément en forme de plaque (12) avec lesdits moyens de préhension (9) en prise avec ledit élément d'ancrage (11).

5. - Equipement (1) selon l'une ou plusieurs des revendications précédentes, **caractérisé par le fait que** lesdits moyens de préhension (9) sont du type pince et sont mobiles entre une position ouverte, afin de permettre l'introduction/l'extraction dudit élément d'ancrage (11) à l'intérieur de ceux-ci, et une position fermée, dans laquelle ils interagissent avec ledit élément d'ancrage (11) afin d'empêcher le déplacement mutuel de ceux-ci.

6. - Equipement (1) selon l'une ou plusieurs des revendications précédentes, **caractérisé par le fait que** ladite enveloppe (6) définit au moins deux parois latérales (7a), une paroi avant (7b) et une paroi de base (7c) délimitant ladite chambre de logement (7), **par le fait que** lesdits moyens d'ancrage (8) sont associés à ladite enveloppe (6) à l'aide de moyens de fixation amovibles (19), et **par le fait que** lesdits moyens de fixation (19) comprennent au moins un premier organe de fixation (19a) associé à chacune desdites parois latérales (7a), à ladite paroi avant (7b) et à ladite paroi de base (7c) et au moins un second organe de fixation (19b) associé audit premier élément en forme de plaque (10) et audit second élément en forme de plaque (12) et apte à fonctionner en coopération avec un premier organe de fixation précité correspondant (19a) pour verrouiller lesdits moyens d'ancrage (8) conjointement avec ladite enveloppe (6).

7. - Equipement (1) selon l'une ou plusieurs des revendications précédentes, **caractérisé par le fait que** ladite enveloppe de confinement (6) est du type d'un sac à dos fait d'un matériau souple.

8. - Equipement (1) selon la revendication 7, **caractérisé par le fait que** ladite unité biomédicale (2) comprend un circuit actionné par fluide (5) pour la circulation sanguine relié audit oxygénateur (O) et à ladite pompe centrifuge (P) et **par le fait que** ladite enveloppe de confinement (6) comprend un compartiment (14) pour loger ledit circuit actionné par fluide (5).

9. - Equipement (1) selon la revendication 8, **caractérisé par le fait que** ladite enveloppe de confinement (6) comprend au moins deux parties (6a, 6b), dont une première partie (6a) définit ladite chambre de logement (7) et une seconde partie (6b) définit ledit compartiment (14), et **par le fait que** ladite enveloppe (6) est du type pouvant être ouvert de manière à déplacer mutuellement lesdites parties (6a, 6b) entre une configuration fermée, dans laquelle ladite chambre de logement (7) et ledit compartiment (14) se font face et sont inaccessibles depuis l'extérieur, et une configuration ouverte, dans laquelle ladite chambre de logement (7) et ledit compartiment (14) sont espacés l'un de l'autre par rapport à ladite configuration fermée et sont accessibles depuis l'extérieur.

10. - Enveloppe (6) pour le transport d'une unité biomédicale (2) comportant un cadre porteur de charge flexible (3) et des moyens de support (4) d'au moins un dispositif biomédical (0, P), ladite enveloppe (6) comprenant au moins une chambre de logement (7) dudit cadre porteur de charge (3) comportant des moyens d'ancrage (8) pouvant être engagés avec le cadre porteur de charge lui-même pour bloquer la position de celui-ci à l'intérieur de ladite chambre de logement (7),
**caractérisée par le fait que** ladite enveloppe (6) est du type souple et lesdits moyens d'ancrage (8) sont du type rigide,
et **par le fait que** lesdits moyens d'ancrage (8) comprennent au moins un premier élément en forme de plaque (10) du type rigide associé à ladite enveloppe de confinement (6) et au moins un élément d'ancrage (11) ayant une forme allongée, associé audit premier élément en forme de plaque (10) et tourné vers l'intérieur de ladite chambre de logement (7), lesdits moyens de préhension (9) étant aptes à intercepter ledit élément d'ancrage (11), et **par le fait que** lesdits moyens d'ancrage (8) comprennent au moins un second élément en forme de plaque (12) du type rigide, associé audit premier élément en forme de plaque (10) et disposé transversalement par rapport à celui-ci, ledit cadre porteur de charge (3) comprenant une première partie (3a) portant lesdits moyens de préhension (9) et une seconde partie (3b), verrouillée conjointement avec ladite première partie (3a), apte à être disposée en appui sur ledit second élément en forme de plaque (12).
